Europäisches Patentamt

European Patent Office

Office européen des brevets

(11) Veröffentlichungsnummer: **0 014 268**
**B1**

(12) **EUROPÄISCHE PATENTSCHRIFT**

(45) Veröffentlichungstag der Patentschrift:
07.10.81

(21) Anmeldenummer: 79200548.0

(22) Anmeldetag: 27.09.79

(51) Int. Cl.³: **C 07 D 213/89**, C 07 D 213/71 //
A01N43/40

(54) Verfahren zur Herstellung von Bis-2-pyridyl-disulfiden.

(30) Priorität: 07.02.79 DE 2904548

(43) Veröffentlichungstag der Anmeldung:
20.08.80 Patentblatt 80/17

(45) Bekanntmachung des Hinweises auf die Patenterteilung:
07.10.81 Patentblatt 81/40

(84) Benannte Vertragsstaaten:
AT BE CH DE FR GB IT LU NL

(56) Entgegenhaltungen:
US-A-3 773 770
US-A-3 954 781

CHEMICAL ABSTRACTS, Vol. 53, 1959, Columbus, Ohio, USA
Comprehensive Inorganic Chemistry, S.C. Bailar et al, 1973, Seite 1418

(73) Patentinhaber: Rütgerswerke Aktiengesellschaft,
Mainzer Landstrasse 217, D-6000 Frankfurt a.Main 1 (DE)

(72) Erfinder: Maurer, Manfred, Dr., Bissersheimer Strasse 23,
D-6719 Kirchheim/Weinstr. (DE)
Erfinder: Orth, Winfried, Dr., Am Schachtelgraben 28,
D-6733 Hassloch/Pfalz (DE)
Erfinder: Fickert, Werner, Dr., Stockacher Strasse 14,
D-6800 Mannheim 61 (DE)

## Verfahren zur Herstellung von Bis-2-pyridyl-disulfiden

Die Erfindung betrifft ein Verfahren zur Herstellung von Bis-2-pyridyl-disulfid oder seinem Bis-N-Oxid durch Oxidation von 2-Mercapto-pyridin oder seinem N-Oxid.

Bis-2-pyridyl-disulfide, insbesondere das Bis-(2-pyridyl-1-oxid)-disulfid, finden eine weitverbreitete Anwendung, insbesondere als antibakterielles und fungizides Mittel in der Landwirtschaft, bei kosmetischen Präparaten, wie z.B. Kopfwaschmitteln, oder zum Schutze von Kunststoffen oder Geweben gegen Pilzbefall (vgl. z.B. CA-PS 501 851, US-PS 2 742 476).

Es ist bekannt, Bis-2-pyridyl-disulfide durch Oxidation von 2-Mercaptopyridinen mit Wasserstoffperoxid herzustellen (vgl. US-PS 2 742 476).

Dieses Verfahren ist aber nur dann gut durchführbar, wenn man die Oxidation innerhalb eines eng begrenzten, genau definierten pH-Wertes vornimmt (vgl. DE-OS 2 519 715). Ein Nachteil dieses Verfahrens ist es aber, den pH-Wert während des gesamten Reaktionsablaufes genau einzuhalten; Abweichungen von ±0,5 ergeben bereits Ausbeuteverluste von mindestens 10–20%.

Wegen der Gefahren, die mit der Handhabung von Peroxiden verbunden sind, und der damit verbundenen kostenintensiven Sicherheitsmassnahmen wird nach einem anderen Verfahren das 2-Mercaptopyridin-1-oxid mit Chlor oder Hypochlorit umgesetzt. Dies gelingt aber nur bei einem pH-Bereich von 4–8 in wirtschaftlich vertretbarer Weise (vgl. DE-OS 2 617 489). Durch das fortwährende Entstehen von Salzsäure während der Oxidation ist dabei aber eine dauernde pH-Wert-Einstellung notwendig.

Aufgabe dieser Erfindung war deshalb ein einfaches, sicheres, allgemein anwendbares und auch technisch geeignetes Verfahren zur Herstellung von Bis-2-pyridyl-disulfid oder seines Bis-N-Oxids, das unter Vermeidung der geschilderten Nachteile die Endprodukte in guter Ausbeute und Reinheit liefert.

Erfindungsgemäss wird diese Aufgabe durch ein Verfahren zur Herstellung von Bis-2-pyridyl-disulfid oder seines Bis-N-Oxids durch Oxidation von 2-Mercapto-pyridin oder seinem N-Oxid gelöst, das dadurch gekennzeichnet ist, dass man die Oxidation im wässerigen Medium mit Alkali- oder Erdalkalichlorit bei einem pH-Wert im Bereich von 3,5 bis 5,5 durchführt.

Als Chlorit kann jedes Alkali- oder Erdalkalichlorit, also z.B. Lithium- oder Kaliumchlorit, oder z.B. Magnesium- oder Calciumchlorit, eingesetzt werden, vorzugsweise wird Natriumchlorit verwendet. Die Zugabe des Chlorits kann in fester Form zu einer wässerigen Lösung oder Suspension der Mercaptopyridine erfolgen. Es kann vorteilhafterweise auch eine Lösung oder Suspension eines Alkali- oder Erdalkalisalzes der Mercaptopyridine eingesetzt werden.

Zweckmässigerweise erfolgt die Zugabe des Chlorits in Form einer wässerigen Lösung, z.B.

als als Handelsprodukt erhältliche 24,2%ige Natriumchloritlösung. Die Reaktion wird vorzugsweise im pH-Bereich von 4,0–5,0 durchgeführt. Im alkalischen Bereich erfolgt keine Reaktion. Man arbeitet zweckmässigerweise bei einer Temperatur von 0–70°C, insbesondere 20–35°C. Das Verfahren ist auch mit Ausgangsprodukten durchführbar, in denen der Pyridinring durch einen oder mehrere Substituenten, z.B. aus der Gruppe Alkyl, Alkoxy oder Halogen, wie z.B. Chlor oder Brom, substituiert ist. In diesen Substituenten besitzt eine Alkylgruppe vorzugsweise 1–4 C-Atome.

Die Zugabe des Oxidationsmittels kann zur wässerigen Lösung oder Suspension, die vorher auf den geeigneten pH-Wert eingestellt wurde, erfolgen; es kann aber auch zuerst das Oxidationsmittel zugegeben und danach der pH-Wert eingestellt werden. Die Zugabe des Oxidationsmittels erfolgt vorzugsweise portionenweise und unter Rühren oder Schütteln des Reaktionsgemisches. Sehr gute Ergebnisse in bezug auf Ausbeute und Reinheit des Reaktionsproduktes erhält man, wenn das Chlorit in stöchiometrischem Überschuss, vorzugsweise bis zu 20%, bezogen auf das Salz des 2-Mercaptopyridins, eingesetzt wird. Zur Vervollständigung der Reaktion ist es zweckmässig, nach beendeter Zugabe des Chlorits die Reaktionsmischung noch etwa 3 bis 5 Stunden bei der Reaktionstemperatur zu rühren.

Die Einstellung des pH-Wertes kann durch Zugabe einer nicht oxidierend wirkenden Mineralsäure oder organischen Säure erfolgen, z.B. mit Salzsäure oder Essigsäure.

Die Herstellung der als Ausgangsmaterial eingesetzten 2-Mercapto-pyridine erfolgt auf bekannte Weise, z.B. aus 2-Chlorpyridin, gegebenenfalls nach vorhergehender Oxidation zum N-Oxid (DE-PS 1 470 162, US-PS 2 951 844), durch Umsetzung mit Alkali- oder Erdalkalisulfid (US-PS 2 686 786, US-PS 3 159 640).

Nach dem erfindungsgemässen Verfahren wird das Disulfid in hohen Ausbeuten und hoher Reinheit erhalten, die ohne zusätzliche Reinigungsoperationen bereits den Reinheitsforderungen für die Weiterverarbeitung, z.B. zu kosmetischen Präparaten, entspricht.

Ein weiterer Vorteil ist es, dass man nach dem erfindungsgemässen Verfahren auch beim Einsatz von je nach der Herstellungsart mehr oder weniger stark verunreinigten Lösungen der Mercaptopyridine gute Ergebnisse erzielt.

Unter Prozentangaben sind Gewichtsprozente zu verstehen. Temperaturangaben beziehen sich auf die Celsiusskala.

Die dünnschichtchromatografischen Analysen wurden an Kieselgelplatten vom Typ F 254/366 der Fa. Woelm, Eschwege, ausgeführt. Als Lösungsmittel wurde Methanol/Chloroform (1/1), als Laufmittel Essigester/Methanol/Eisessig (75/ 20/ 5) verwendet. Die Auftragsmenge betrug 50 $\gamma$.

## Beispiele

### Beispiel 1

2440 g (2000 ml) einer wässerigen Lösung, die neben Kochsalz 12,3% Natrium-2-mercapto-pyridin-1-oxid (298 g = 2 Mol) enthält, werden nach dem Verdünnen mit 350 ml Wasser unter Rühren mit etwa 170 ml konz. Salzsäure auf pH 4,5 gestellt. Nach Abkühlen auf 20°C gibt man innerhalb von 60 Min. portionenweise unter gutem Rühren 68 g 80proz. Natriumchlorit (fest) (0,6 Mol) zu. Die Temperatur steigt dabei auf 35°C und kann durch äussere Kühlung im Bereich von 20–35°C gehalten werden. Man lässt 5 Stunden nachrühren, saugt das ausgefallene Bis-(2-pyridyl-1-oxid)-disulfid ab und wäscht mit 1000 ml Wasser. Nach dem Trocknen bei 100–110°C erhält man 219 g (= 87% d.Th.) Bis-(2-pyridyl-1-oxid)-disulfid vom Schmelzpunkt: 203–205°C. Das Dünnschichtchromatogramm (DC) ist einheitlich.

### Beispiel 2

Man arbeitet wie in Beispiel 1 beschrieben, verwendet jedoch anstelle von festem Natriumchlorid 224 g (0,6 Mol) einer 24,2%igen wässerigen Lösung.

Schmelzpunkt: 203–205°C.
Dünnschichtchromatogramm: einheitlich.
Ausbeute: 232 g (92% d.Th.).

### Beispiel 3

2920 g einer wässerigen Lösung, die etwa 8% Natriumchlorid, etwa 12% Natriumacetat und 10,2% Natrium-2-mercapto-pyridin-1-oxid (298 g = 2 Mol) enthält, wird unter gutem Rühren mit konz. Salzsäure auf pH 5,0 eingestellt. Zu der gut gerührten Mischung tropft man bei einer Temperatur von 20–35°C/224 g innerhalb 1 Stunde einer 24,2%igen, wässerigen Natriumchloritlösung (0,6 Mol). Man lässt 4 Stunden nachrühren, saugt den ausgefallenen Niederschlag ab, wäscht mit 1000 ml Wasser und trocknet bei 100–110°C.
Ausbeute an Bis-(2-pyridyl-1-oxid)-disulfid: 230,5 g (91,8% d.Th.).
Schmelzpunkt: 203–205°C.
Dünnschichtchromatogramm: einheitlich.

### Beispiele 4–8

In der folgenden Tabelle ist eine Übersicht gegeben über Beispiele unter Verwendung wässeriger Chloritlösungen nach Beispiel 2, jedoch unter Variation von pH-Wert und Reaktionstemperatur.

| Beispiel | pH-Wert | Temp. (°C) | Ausbeute (% d.Th.) |
|----------|---------|------------|--------------------|
| 4 | 3,5 | 20–35 | 84,8 |
| 5 | 4,0 | 20–35 | 90,9 |
| 6 | 5,5 | 20–35 | 89,1 |
| 7 | 4,5 | 0–10 | 91,8 |
| 8 | 4,5 | 60–65 | 84,3 |

### Beispiel 9

Man arbeitet wie unter Beispiel 2 beschrieben, verwendet jedoch anstelle von Salzsäure Essigsäure zum Einstellen auf pH 5,5.
Ausbeute: 225 g (89,2% d.Th.).
Schmelzpunkt: 203–205°C.
Dünnschichtchromatogramm: einheitlich.

### Beispiel 10

2440 g (2000 ml) einer wässerigen Lösung wie im Beispiel 1, die 12,3% Natrium-2-mercaptopyridin-1-oxid enthält, werden bei Raumtemperatur (25°C) mit 224 g (0,6 Mol) einer 24,2%igen Natriumchloritlösung versetzt. Unter äusserer Kühlung und einem Temperaturanstieg auf 35°C tropft man innerhalb 60 Minuten 170 ml konz. Salzsäure zu. Dabei stellt sich ein pH-Wert von 4,5 ein. Während der Reaktion beginnt das Bis-(2-pyridyl-1-oxid)-disulfid auszufallen. Man rührt 5 Stunden nach und arbeitet wie in Beispiel 1 beschrieben auf.
Ausbeute: 234 g (92,8% d.Th.).
Schmelzpunkt: 203–205°C.
Dünnschichtchromatogramm: einheitlich.

### Beispiel 11

In eine gerührte Mischung aus 222 g (2 Mol) 2-Mercaptopyridin in 1500 ml Wasser, 50 ml Essigsäure sowie einigen Tropfen eines Netzmittels tropft man in 15–30 Minuten bei 25°C 210 g (0,56 Mol) einer 24,2proz. Natriumchlorit-Lösung. Nachdem man die Suspension noch 2 Stunden bei Raumtemperatur nachgerührt hat, saugt man vom Produkt ab, wäscht mit Wasser nach und trocknet bei max. 40°C im Vakuum.
Ausbeute an 2,2'-Bispyridyl-disulfid: 202 g, (92% d.Th.).
Schmelzpunkt: 55–56°C.
Dünnschichtchromatogramm: einheitlich.

### Patentansprüche

1. Verfahren zur Herstellung von Bis-2-pyridyl-disulfid oder seinem Bis-N-Oxid durch Oxidation von 2-Mercaptopyridin oder seinem N-Oxid, dadurch gekennzeichnet, dass man die Oxidation im wässerigen Medium mit Alkali- oder Erdalkalichlorit bei einem pH-Wert im Bereich von 3,5–5,5 durchführt.

2. Verfahren nach Anspruch 1, dadurch gekennzeichnet, dass man bei einem pH-Wert von 4,0–5,0 arbeitet.

3. Verfahren nach einem der Ansprüche 1 bis 2, dadurch gekennzeichnet, dass man die Oxidation bei einer Temperatur von 0–70°C durchführt.

4. Verfahren nach einem der Ansprüche 1 bis 3, dadurch gekennzeichnet, dass man die Oxidation bei einer Temperatur von 20–35 °C durchführt.

5. Verfahren nach einem der Ansprüche 1 bis 4, dadurch gekennzeichnet, dass man als Oxidationsmittel eine wässerige Alkali- oder Erdalkalichloritlösung verwendet.

6. Verfahren nach einem der Ansprüche 1 bis 5, dadurch gekennzeichnet, dass man als Oxidationsmittel Natriumchlorit verwendet.

7. Verfahren nach einem der Ansprüche 1 bis 6, dadurch gekennzeichnet, dass man das Alkalichlorit im bis zu 20%igen stöchiometrischen Überschuss einsetzt.

## Claims

1. A process for the preparation of bis-2-pyridyl-disulfide or bis-(2-pyridyl-1-oxide)-disulfide by oxidation of 2-mercapto-pyridine or its N-oxide, characterized in that the oxidation is made in an aqueous media by an alkali metal or alkaline earth metal chlorite at a pH in the range of 3,5 to 5,5.

2. The process of claim 1 wherein the pH is 4,0 to 5,0.

3. The process of claim 1 or 2 wherein the oxidation is run at the temperature of 0 to 70°C.

4. The process of one of the claims 1 to 3 wherein the oxidation is run at a temperature of 20 to 35°C.

5. The process of one of the claims 1 to 4 wherein an aqueous solution of an alkali metal or alkaline earth metal chlorite is used as oxidation agent.

6. The process of one of the claims 1 to 5 wherein sodium chlorite is used as oxidation agent.

7. The process of one of the claims 1 tu 6 wherein the alkali chlorite is used in a stoichiometric excess up tu 20%.

## Revendications

1. Procédé de préparation du bis-2-pyridyldisulfure ou de son bis-N-oxyd par oxydation de la 2-mercapto-pyridine ou de son N-oxyde, caractérisé par le fait que l'on effectue l'oxydation en milieu aqueux par un chlorite alcalin ou alcélino-terreux à une valeur de pH dans le domaine de 3,5 à 5,5.

2. Procédé selon la revendication 1 caractérisé par le fait que l'on opère à une valeur de pH de 4,0 à 5,0.

3. Procédé selon la revendication 1 ou 2, caractérisé par le fait que l'on effectue l'oxydation à une température de 0 à 70°C.

4. Procédé selon l'une des revendications 1 à 3, caractérisé par le fait que l'on effectue l'oxydation à une température de 20 à 35°C.

5. Procédé selon l'une des revendications 1 à 4, caractérisé par le fait que l'on utilise comme oxydant une solution aqueuse alcaline de chlorite alcalin ou alcalino-terreux.

6. Procédé selon l'une des revendications 1 à 5, caractérisé par le fait que l'on utilise comme oxydant le chlorite de sodium.

7. Procédé selon l'une des revendications 1 à 6, caractérisé par le fait que l'on introduit un chlorite alcalin avec un excès stoechiométrique allant jusqu'à 20%.